# EUROPEAN PATENT SPECIFICATION

(11) **EP 3 887 813 B1**
(45) Date of publication and mention of the grant of the patent: **10.12.2025**
(21) Application number: 19956862.7
(22) Date of filing: 24.12.2019
(51) Int. Cl.: G01N 27/30, G01N 27/327, G01N 33/94

(54) **BIOSENSOR OBTAINED FROM ALGAE BASED CARBON MATERIALS**
BIOSENSOR AUS KOHLENSTOFFMATERIALIEN AUF ALGENBASIS
BIOCAPTEUR OBTENU À PARTIR DE MATÉRIAUX CARBONÉS À BASE D'ALGUES

(30) Priority: 17.12.2019 TR 201920423
(43) Date of publication of application: 06.10.2021
(73) Proprietor: Ankara Üniversitesi Rektörlügü, 06100 Ankara (TR)
(72) Inventor: SINA , Ali, 06100 Ankara (TR); ÖZKAN, Sibel Aysil, 06100 Ankara (TR); BILGE, Selva, 06100 Ankara (TR); KARADAS BAKIRHAN, Nurgül, 06018 Keçiören/Ankara (TR); DONAR, Yusuf Osman, 06100 Ankara (TR)
(74) Representative: Kaya, Erdem
(86) International application number: PCT/TR2019/000083
(87) International publication number: WO 2021/126095

(56) References cited:
- CN-A- 110 487 860
- CN-A- 110 487 860
- US-A1- 2017 237 135
- US-A1- 2017 237 135
- DEMIR ERSIN ET AL: "VOLTAMMETRIC DETERMINATION OF VARDENAFIL ON MODIFIED ELECTRODES CONSTRUCTED BY GRAPHITE, METAL OXIDES AND FUNCTIONALIZED MULTI-WALLED CARBON NANOTUBES", REVUE ROUMAINE DE CHIMIE, vol. 64, no. 1, 1 January 2019 (2019-01-01), RO, pages 45 - 54, XP055837253, ISSN: 0035-3930, DOI: 10.33224/rrch.2019.64.1.04
- WANG TAO ET AL: "VOLTAMMETRIC DETERMINATION OF VARDENAFIL ON MODIFIED ELECTRODES CONSTRUCTED BY GRAPHITE, METAL OXIDES AND FUNCTIONALIZED MULTI-WALLED CARBON NANOTUBES", NEW JOURNAL OF CHEMISTRY, vol. 42, no. 8, 1 January 2018 (2018-01-01), GB, pages 6515 - 6524, XP055837254, ISSN: 1144-0546, DOI: 10.1039/C8NJ00953H
- BILGE SELVA ET AL: "Green synthesis of carbon based biosensor materials from algal biomass for the sensitive detection of vardenafil", JOURNAL OF ELECTROANALYTICAL CHEMISTRY, ELSEVIER, AMSTERDAM, NL, vol. 871, 31 May 2020 (2020-05-31), XP086226014, ISSN: 1572-6657, [retrieved on 20200531], DOI: 10.1016/J.JELECHEM.2020.114286
- MARIA-MAGDALENA TITIRICI ET AL: "Sustainable carbon materials", CHEMICAL SOCIETY REVIEWS, vol. 44, no. 1, 10 October 2014 (2014-10-10), UK, pages 250 - 290, XP055364788, ISSN: 0306-0012, DOI: 10.1039/C4CS00232F
- MARIA-MAGDALENA TITIRICI ET AL: "Sustainable carbon materials", CHEMICAL SOCIETY REVIEWS, vol. 44, no. 1, 10 October 2014 (2014-10-10), UK, pages 250 - 290, XP055364788, ISSN: 0306-0012, DOI: 10.1039/C4CS00232F
- DEMIR ERSIN, BOZAL-PALABIYIK BURCIN, USLU BENGI, INAM RECAI: "VOLTAMMETRIC DETERMINATION OF VARDENAFIL ON MODIFIED ELECTRODES CONSTRUCTED BY GRAPHITE, METAL OXIDES AND FUNCTIONALIZED MULTI-WALLED CARBON NANOTUBES", REVUE ROUMAINE DE CHIMIE, EDITURA ACADEMIEI ROMANE, RO, vol. 64, no. 1, 1 January 2019 (2019-01-01), RO, pages 45 - 54, XP055837253, ISSN: 0035-3930, DOI: 10.33224/rrch.2019.64.1.04
- WANG TAO, LIU XIQING, MA CHANGCHANG, LIU YANG, DONG HONGJUN, MA WEI, LIU ZHI, WEI MAOBIN, LI CHUNXIANG, YAN YONGSHENG: "A two step hydrothermal process to prepare carbon spheres from bamboo for construction of core–shell non-metallic photocatalysts", NEW JOURNAL OF CHEMISTRY, ROYAL SOCIETY OF CHEMISTRY, GB, vol. 42, no. 8, 1 January 2018 (2018-01-01), GB, pages 6515 - 6524, XP055837254, ISSN: 1144-0546, DOI: 10.1039/C8NJ00953H

## Description

### Technical Field of the Invention

The invention relates to a biosensor-oriented use of carbon materials, which are obtained as a result of nanotube-catalysed seconder hydrothermal carbonization process.

### State of art

In the literature, there are many biomass conversion methods such as pyrolysis, liquefaction, gasification, combustion and hydrothermal carbonization method. Among these methods, hydrothermal carbonization method gained high prominence in recent years, compared to other methods. The reason behind it is that hydrothermal carbonization method contains many advantages. Among these advantages are that the synthesis can be carried out in aqueous phase and toxic solvents are not necessary, solvents can be recovered during the process, the process is composed of eco-friendly process steps, it is possible to have high amount of product yield, it is a method that is suitable for scaling and that enables a large scale synthesis, it is possible to obtain materials with high surface area, working conditions are at low temperature, additional cooling systems that are required to be contained in the other process is not necessary in synthesis, the obtained products contain groups with various functions (therefore a test phase such as surface functioning).

Hydrothermal carbonization process can be basically defined as the whole of heterogeneous reactions of the biomass formed in a closed contained under 100°C temperature and 1 bar pressure.

All materials that are formed of organisms contain biomass, which is the primary of renewable energy sources and it is a renewable energy source with the highest application potential. The most important feature of renewable energy sources is that they can be formed in the same amount again and same level of energy can be obtained after they are used by undergoing a specific process.

Biomass can be defined as biological mass which is formed as a result of conversation of solar energy to chemical energy by green plants through photosynthesis and organic material sources such as any related green plant and animal waste.

In the state of art, hydrothermal carbonization method is applied once to any materials having biomass characteristics. The formed material is charred by undergoing a significant transformation compared to its input state and its surface characteristics change considerably. The factor which causes this transformation is the use of water as a solution in hydrothermal method and function groups are formed on the surface of the material which is charred under a specific temperature and pressure. This function groups have a great importance in electrochemical applications because function groups which are formed on the surface increase determination rate of the material which will undergo an electrochemical analysis by increasing electrical conductivity. In the present invention, the method is used twice consecutively through catalysis. Therefore, the structure of algae based carbon material which is obtained as a result of the hydrothermal carbonization applied for the second time is clarified through various analysis methods and it was determined that it is more conductive and that the surface function groups are more than the carbon material obtained as a result of the first HTC process.

It was found out that there is not any application concerning the use of electrochemical use of carbon materials obtained as a result of hydrothermal carbonization process that is applied for the second time upon catalysis as a result of detailed literature researches. Use of the inventive material is novel around the world.

Demir Ersin ET AL: "Voltammetric determination and vardenafil on modified electrodes made of graphite, metal oxides and functionalized multi-walled carbon nanotubes" study attracts attention in the relevant technical field. This study explored the voltammetric behavior of vardenafil using three electrochemical techniques: square wave stripping voltammetry (SWSV), differential pulse stripping voltammetry (DPSV), and cyclic voltammetry (CV). Modified electrodes, including graphite, metal oxide, and functionalized multi-walled carbon nanotubes (MWCNT), were utilized for the investigations. This study explored the voltammetric behavior of vardenafil using three electrochemical techniques: square wave stripping voltammetry (SWSV), differential pulse stripping voltammetry (DPSV), and cyclic voltammetry (CV). Modified electrodes, including graphite, metal oxide, and functionalized multi-walled carbon nanotubes (MWCNT), were utilized for the investigations.

Another study in the relevant technical field is Wang Tao Et Al: "A two step hydrothermal process to prepare carbon spheres from bamboo for construction of core-shell non-metallic photocatalysts" article. This study presents a two-step hydrothermal carbonization (HTC) process to convert bamboo biomass into carbon spheres at temperatures between 180°C and 200°C. These carbon spheres show promise as electrode materials due to their excellent electrical conductivity and chemical stability. The biochar spheres (BS) are further modified with protonated g-C3N4 to create non-metallic photocatalysts with a core-shell structure. In these BS@g-C3N4 photocatalysts, protonated g-C3N4 sheets act as the main photocatalyst, while the biochar spheres serve as both a reservoir and sensitizer. They exhibit exceptional light absorption across various wavelengths due to their unique carbon cluster properties. This approach offers a green and environmentally friendly method to convert raw biomass into stable carbon materials, addressing challenges in controlling biochar morphology.

Another work in the technique is the patent application with publication number CN110487860. This invention outlines a method for preparing a seaweed-based biomass carbon (SDBC) / nano-gold (AuNPs) modified electrode and its application in detecting the content of rutin. The invention presents an efficient and cost-effective method for preparing a modified electrode and its application in rutin content detection, offering improved sensitivity and a wide linear range for analysis.

Another work in the technique is the patent application with publication number US2017/237135. The document suggests the need for an electrode material that can operate under pH-neutral conditions, along with an electrode and battery incorporating this material. An embodiment of the disclosure introduces an electrode made of plant-derived porous carbon material capable of catalyzing oxygen reduction. Similarly, there is an embodiment of an electrode material featuring this plant-derived porous carbon material. Additionally, the disclosure presents a battery with a positive electrode that incorporates plant-derived porous carbon material for oxygen reduction catalysis. These materials, electrodes, and batteries leverage plant-derived porous carbon to effectively catalyze oxygen reduction under pH-neutral conditions. Importantly, they do not rely on enzymes, reducing limitations related to the operating environment, which may vary depending on the battery's structure. Another study in the relevant technical field is Maria-Magdalena Titirici Et Al: "Suistainable carbon materials" article. The review explores recent advancements in sustainable carbon material production and their applications in critical environmental and energy-related fields.

### Technical problems aimed to be solved by the invention

The object of the invention is to develop an electrode material synthesizing method concerning an affordable and simple-to-use biosensor system for detecting vardenafil active ingredient, which is included in erectile dysfunction drug class, through differential pulse voltameter in a biological media such as serum, urine and blood. In the literature, hydrothermal carbonization method is applied as two cycles for the first time and the product which was formed by nanotube catalysis is used as an electrode material. The carbon material which is obtained by applying a second hydrothermal carbonization process to algae based carbon material which was obtained as a result of hydrothermal carbonization (HTC) method that has been used in experimental studies recently plays the most crucial part in determining vardenafil active ingredient in a fast and correct way. The active ingredient can be easily determined when this material is used in electrode modification. Furthermore, algae based carbon material, which is obtained as a result of the first HTC process, is catalyzed by a multi-walled carbon nanotube while it is treated in the second HTC process in order to improve the produced biosensor and the conductivity of the material is increased. Thus, the electric signal, which shows the determination of active pharmaceutical ingredient, is increased.

### Description of the invention

The invention directly relates to renewable energy, biochemistry and biotechnology fields due to the fact that hydrothermal carbonization method within the scope of the invention is an important biomass transformation method and algae which is used as a carbon resource is a biological species. The invention is defined by a method for the electrochemical determination of vardenafil active indgredient in biological media according to claim 1, a method of improving the sensing surface of an electrode according to claim 3 and the use of algae-based carbon material according to claim 4.

### Description of Figures

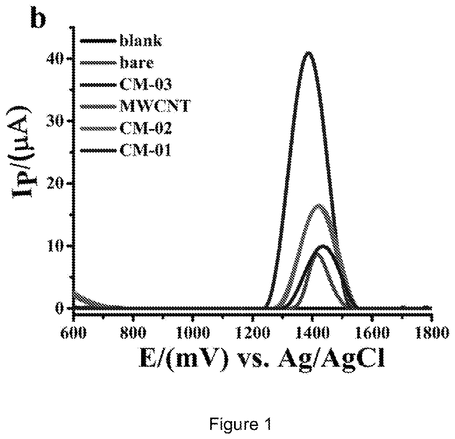

Figure 1: Differential pulse voltammograms (Current- potential)

### Detailed description of the invention:

An autoclave system with the following specifications is used in the experiment. First, the algae species, Cystoseira, was subjected to hydrothermal carbonization process for 8 hours in an aqueous medium under 180°C temperature. The biomass resource was dried and dehumidified under 100 °C and sieved to 150 µm before beginning of the tests. This phase contains the application of hydrothermal carbonization process without adding any difference and novelty. In the final phase, the carbon product which was obtained from the first HTC process was subjected to the second HTC process for 8 hours under 250 °C in a very little amount again in an aqueous medium. This final phase is the phase having the characteristics of the invention and degree of graphitization of the product which was formed as a result of the second HTC process increased, which in turn increased the electrical conductivity (this result was obtained based on the analysis conducted on samples). So as to observe effects of the second HTC process in a detailed manner, HTC process was conducted without further conducting catalysis on the carbon, which was obtained upon the first HTC process.

### Test systems

### Autoclave system

The autoclave system, which is used in hydrothermal carbonization is made of stainless steel and has an internal volume of 300 ml. The device comprising its own oven, which can control the temperature during the test with a thermocouple system, has a temperature and pressure indicator of 400°C temperature and of 200 bar (3000 PSI). Maximum 350 °C and 138 bar (2000 PSI) is determined as the safe working conditions of the system.

### Biosensor system

A system with three electrodes being reference electrode, counter electrode and working electrode is used in biosensor tests. Ag/AgCl is used as the reference electrode and Platin is used as the counter electrode. Finally, a vitreous electrode is selected as the working electrode and left to dry by applying modification with synthesized materials through dropping method. Biosensor tests are conducted with the aforementioned three-electrode system.

Although there are studies in which various materials are synthesized through hydrothermal carbonization method and used in biosensor field, in this study algae reserved in Turkey are selected as a carbon resource and they are synthesized by the application of secondary hydrothermal carbonization method contrary to the method which was worked in the literature for the first time and they are used for determining vardenafil that is included in erectile dysfunction drug active ingredient, which was not studied in biosensor applications (which was made using modified electrode method) before. The invention has the qualification of being studied in the literature for the first time.

Differential pulse voltammograms in the presence of 5×10⁻⁵ Vardenafil active ingredient in pH 1.0 H₂SO₄ buffer is shown in Figure 1. CM-03 increases peak current more compared to CM-01, CM-02 and MWCNT materials and enabled peak potential to slip.

Synthesized sensor materials are left to dry after they are dropped by dropping method on vitreous carbon electrode surface. Then, they are dipped into the cell containing active pharmaceutical ingredient and pH 1.0 H₂SO₄ solution and they are measured.

Upon the examination of the result of the test which was conducted within the scope of the invention, it was concluded that CM-03 having the qualification of a biosensor material can be used over and over again. Thus, it is foreseen that there shall not be any serious negativity concerning life cycle of the invention in the field of technology.

Carbon materials, which are obtained as a result of the invention, have the potential to be used in various electrochemical applications such as in super capacitor and capacitor field, in the development of electrocoating-based solar cells, photo-electrochemistry and energy storage fields.

Erectile dysfunction is used for determining vardenafil active ingredient, which is included in active pharmaceutical ingredient class, in biological media in the invention. In the studies to be conducted in the short term, it can be used for determining an active pharmaceutical ingredient used in another illness instead of vardenafil.

Brine algae, which are found abundant as a reserve, can be easily supplied due to the abundance of water resources in Turkey and they are at no cost. The fact that algae, which are abundant in Turkey, are to be evaluated in this way shows that they are advantageous in commercial terms.

The invention is easy to illustrate and present as it is of a simple and functional system.

Erectile dysfunction active ingredient, vardenafil, could be electrochemically determined successfully serum and urinary medium under laboratory conditions. The invention shall contribute to the fields of health and pharmacy when its use in large-scale applications is evaluated.

## Claims

1. A method for the electrochemical determination of vardenafil active ingredient in biological media such as serum and urine, **characterized in that** an electrode modification material used in the said electrochemical method is algae-based carbon obtained by a first step of subjecting an algae species to a first hydrothermal carbonization process (HTC process),
**characterized in that**
that first step is followed by a second step of subjecting in the product of the first step to a second hydrothermal carbonization process which is catalysed by carbon nanotubes.

2. A method according to claim 1, **characterized in that** the algae in said algae-based carbon is the genus Cystoseria.

3. A method for improving the sensing surface and increasing the performance of an electrode used in the electrochemically sensitive and selective determination of vardenafil active ingredient in biological media such as serum and urine, **characterized in that** the said method is applied in two cycles as hydrothermal carbonization process and includes the following process steps,
- Drying of Cystoseira algae species at temperature of 100°C and removing moisture and sieving to 150 micrometers,
- After the mentioned process step, subjecting the Cystoseira algae species to the first hydrothermal carbonization process at 180°C for 8 hours and obtaining the carbon product,
- Applying the second hydrothermal carbonization process to the resulting carbon product in a carbon nanotube medium at 250°C for 8 hours.

4. Use of algae-based carbon material as an electrode material in the development of electroplating based solar cells in the supercapacitor and capacitor fields, photo-electrochemistry and energy storage fields, **characterized in that** the algae-based carbon material is
obtained by a first step of subjecting an algae species to first hydrothermal carbonization process (HTC process), wherein that first step is followed by a second step of subjecting the product of the first step to a second hydrothermal carbonization process which is catalysed by carbon nanotubes.

## Patentansprüche

1. Verfahren zur elektrochemischen Bestimmung des Wirkstoffs Vardenafil in biologischen Medien wie Serum und Urin, **dadurch gekennzeichnet, dass** als Elektrodenmodifikationsmaterial in dem elektrochemischen Verfahren Kohlenstoff auf Algenbasis verwendet wird, der in einem ersten Schritt durch einen ersten hydrothermalen Karbonisierungsprozess (HTC-Prozess) aus einer Algenart gewonnen wird, **dadurch gekennzeichnet, dass** auf diesen ersten Schritt ein zweiter Schritt folgt, bei dem das Produkt des ersten Schritts einem zweiten hydrothermalen Karbonisierungsprozess unterzogen wird, der durch Kohlenstoffnanoröhren katalysiert wird.

2. Verfahren nach Anspruch 1, **dadurch gekennzeichnet, dass** die Algen in dem Kohlenstoff auf Algenbasis zur Gattung Cystoseria gehören.

3. Verfahren zur Verbesserung der Sensorfläche und zur Steigerung der Leistungsfähigkeit einer Elektrode, die bei der elektrochemisch sensitiven und selektiven Bestimmung des Vardenafil-Wirkstoffs in biologischen Medien wie Serum und Urin verwendet wird, **dadurch gekennzeichnet, dass** das Verfahren in zwei Zyklen als hydrothermischer Karbonisierungsprozess angewendet wird und die folgenden Verfahrensschritte umfasst:
- Trocknung von Cystoseira-Algenarten bei einer Temperatur von 100°C, und Entfernung von Feuchtigkeit und Siebung auf 150 Mikrometer,
- nach dem genannten Verfahrensschritt, Unterziehung der Algenart Cystoseira einem ersten hydrothermalen Karbonisierungsprozess bei 180°C für 8 Stunden und Gewinnung des Kohlenstoffprodukts,
- Anwendung des zweiten hydrothermalen Karbonisierungsprozesses auf das resultierende Kohlenstoffprodukt in einem Kohlenstoffnanoröhrenmedium bei 250°C für 8 Stunden.

4. Verwendung von Kohlenstoffmaterial auf Algenbasis als Elektrodenmaterial bei der Entwicklung von galvanisch basierten Solarzellen im Bereich von Superkondensatoren und Kondensatoren, in den Bereichen Photoelektrochemie und Energiespeicherung, **dadurch gekennzeichnet, dass** das Kohlenstoffmaterial auf Algenbasis in einem ersten Schritt durch einen ersten hydrothermalen Karbonisierungsprozess (HTC-Prozess) aus einer Algenart gewonnen wird, wobei auf diesen ersten Schritt ein zweiter Schritt folgt, in dem das Produkt des ersten Schritts einem zweiten hydrothermalen Karbonisierungsprozess unterzogen wird, der durch Kohlenstoffnanoröhren katalysiert wird.

## Revendications

1. Une méthode pour la détermination électrochimique du principe actif vardénafil dans des milieux biologiques tels que le sérum et l'urine, **caractérisée en ce qu'**un matériau de modification d'électrode utilisé dans ladite méthode électrochimique est du carbone à base d'algues obtenu par une première étape consistant à soumettre une espèce d'algues à un premier processus de carbonisation hydrothermique (processus HTC), **caractérisé en ce que** ladite première étape est suivie d'une deuxième étape consistant à soumettre le produit de la première étape à un deuxième processus de carbonisation hydrothermique catalysé par des nanotubes de carbone.

2. Une méthode selon la revendication 1, **caractérisée en ce que** les algues dans ledit carbone à base d'algues appartiennent au genre Cystoseria.

3. Une méthode pour améliorer la surface de détection et augmenter les performances d'une électrode utilisée dans la détermination électrochimiquement sensible et sélective du principe actif du vardénafil dans des milieux biologiques tels que le sérum et l'urine, **caractérisée en ce que** ladite méthode est appliquée en deux cycles sous forme de processus de carbonisation hydrothermique et comprend les étapes suivantes du processus
- Sécher les algues de l'espèce Cystoseira à une température de 100 °C et éliminer l'humidité et tamiser à 150 micromètres,
- Après l'étape du processus mentionnée, soumettre l'espèce d'algue Cystoseira au premier processus de carbonisation hydrothermique à 180 °C pendant 8 heures et obtenir le produit carboné,
- Application du deuxième processus de carbonisation hydrothermique au produit carboné résultant dans un milieu de nanotubes de carbone à 250 °C pendant 8 heures.

4. Utilisation d'un matériau carboné à base d'algues comme matériau d'électrode dans le développement de cellules solaires à base d'électroplacage dans les domaines des supercondensateurs et des condensateurs, les domaines de la photo-électrochimie et du stockage d'énergie, **caractérisée en ce que** le matériau à base de carbone issu d'algues est obtenu par une première étape consistant à soumettre une espèce d'algues à un premier processus de carbonisation hydrothermique (processus HTC), dans lequel cette première étape est suivie d'une deuxième étape consistant à soumettre le produit de la première étape à un deuxième processus de carbonisation hydrothermique catalysé par des nanotubes de carbone.
